(19) **Europäisches Patentamt / European Patent Office / Office européen des brevets**

(11) **EP 4 703 341 A1**

(12) # EUROPEAN PATENT APPLICATION

(43) Date of publication:
04.03.2026 Bulletin 2026/10

(21) Application number: 24198269.3

(22) Date of filing: 03.09.2024

(51) International Patent Classification (IPC):
*C07C 29/132* (2006.01)   *C07C 31/20* (2006.01)
*B01J 23/46* (2006.01)   *C07C 29/60* (2006.01)

(52) Cooperative Patent Classification (CPC):
(C-Sets available)
**C07C 29/60; B01J 21/18; B01J 23/462;
B01J 35/392; B01J 35/40; B01J 35/615;
C07C 29/132** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Avantium Knowledge Centre B.V.
1014 BV Amsterdam (NL)**

(72) Inventors:
- **ESMAEILI, Faezeh
  1014 BV Amsterdam (NL)**
- **MCKAY, Benjamin
  1014 BV Amsterdam (NL)**
- **RUSSELL, Scott
  1014 BV Amsterdam (NL)**

(74) Representative: **Avantium Intellectual Property
PO Box 169
8330 AD Steenwijk (NL)**

(54) **CATALYST FOR PREPARING ETHYLENE GLYCOL AND/OR PROPYLENE GLYCOL**

(57) Process for the preparation of ethylene glycol and/or propylene glycol by reacting a carbohydrate source with hydrogen in the presence of tungsten compound and a heterogeneous catalyst comprising a metal of groups 8, 9 and 10 on a support which heterogeneous catalyst has (a) an active metal surface area (AMSA) of from 4.0 to 8.0 $m^2$/g, as determined by hydrogen chemisorption, and (b) a ratio of the percentage of the adsorbed ammonia that is desorbed at a temperature range of 100 °C to the temperature of the first peak (% DA100-FP) to the AMSA (% DA100-FP/AMSA) of from 0.35 to 8.0, and (c) $7.18 \times AMSA - \frac{\%(DA100-FP)}{AMSA}$ is at least 26.38, and process of selecting such heterogeneous catalyst.

EP 4 703 341 A1

(52) Cooperative Patent Classification (CPC): (Cont.)

C-Sets
**C07C 29/132, C07C 31/202;**
**C07C 29/60, C07C 31/205**

**Description**

**Introduction**

The present invention relates to a process for the preparation of ethylene glycol and/or propylene glycol from a carbohydrate source. The invention also relates to a process for selecting a heterogeneous catalyst for use in such process.

**Background of the invention**

Alkylene glycols, such as ethylene glycol and propylene glycol, are currently mainly produced from fossil fuels. They represent interesting chemicals that find application in the preparation of polyesters, such as poly(alkylene terephthalate), poly(alkylene naphthenate) or poly(alkylene furandicarboxylate). Further applications of alkylene glycols, in particular ethylene glycol, include their use as anti-freeze. By enabling the preparation of such chemicals from sustainable resources, the dependence of fossil fuel resources is reduced. Hence, there is a growing interest in the production of alkylene glycols, such as ethylene glycol, from sustainable resources.

In US 7,960,594 a process is described wherein ethylene glycol is produced from cellulose. This process involves catalytic degradation and hydrogenation reactions under hydrothermal conditions. More in particular, the process is carried out by contacting cellulose at elevated temperature and pressure with a catalyst system comprising two sorts of active components in the presence of hydrogen. The first active component comprises tungsten or molybdenum in its metallic state or in the form of its carbide, nitride or phosphide. The second component is selected from the hydrogenation metals from Groups 8, 9 and 10 of the Periodic Table of Elements, and includes cobalt, nickel, ruthenium, rhodium, palladium, iridium and platinum. In experiments the catalysts compounds were used on a carrier, such as activated carbon. Moreover, it appears that the reaction conditions that result in satisfactory yields include a temperature of 220 - 250 °C and a hydrogen pressure of 3 to 7 MPa. Although it is reported that initially the yield of ethylene glycol can be high, it also appears that when the reaction is continued for a prolonged period the ethylene glycol yield reduces significantly.

This reaction has been further studied for catalyst systems that contain nickel and tungsten on a carrier. There it has been found that nickel and tungsten are leached into the solution during the reaction, which accounts for the gradual deterioration of the catalyst performance (cf. Na Ji et al., ChemSusChem, 2012, 5, 939-944). The leaching of tungsten and other metals has been confirmed in the study reported in M. Zheng et al., Chin. J. Catal., 35 (2014) 602-613. The latter document also discloses that in addition to ethylene glycol different by-products are obtained, including erythritol, glycerol, mannitol and sorbitol.

US 2011/0312488 describes a catalyst system for the generation of alkylene glycols from a carbohydrate as a potential alternative for a catalyst comprising the metal components in the elemental state; this catalyst system comprises at least one metal with an oxidation state of at least +2. More in particular this US application discloses a catalyst system comprising a first metal component with an oxidation state of at least +2 and a hydrogenation component. The hydrogenation component can be selected from a wide range of metals in any oxidation state, including in the elemental state. The hydrogenation component may in particular comprise an active metal component selected from the group consisting of Pt, Pd, Ru, Rh, Ni, Ir and combinations thereof. The first metal component may also be selected from a range of metals, but in particular the compounds comprising the first metal component may be selected from the group comprising tungstic acid, molybdic acid, ammonium tungstate, ammonium metatungstate, ammonium paratungstate, tungstate compounds comprising at least one Group 1 or 2 element, metatungstate compounds comprising at least one Group 1 or 2 element, paratungstate compounds comprising at least one Group 1 or 2 element, tungsten oxides, heteropoly compounds of tungsten and various salts and oxides of molybdenum, niobium, vanadium, zirconium, titanium and chromium. The catalyst system according to US2011/0312488 is stated to improve the selectivity to ethylene glycol and propylene glycol, with a reduced production of butane diols. The ethylene glycol generation is shown in some experiments, indicating that ammonium metatungstate is the preferred first metal component and that as preferred hydrogenation component platinum or nickel may be used. The use of nickel-containing catalyst systems results in the highest yields of ethylene glycol and optionally propylene glycol.

In the above-mentioned article of M. Zheng et al., Chin. J. Catal., 35 (2014) 602-613, the conclusion is drawn that tungsten acid-based catalysts are the most promising candidates for future commercialization of the cellulose-to-ethylene-glycol process. A hydrogenation component is added to such tungsten acid-based catalysts. Examples include ruthenium on activated carbon, but Raney nickel is considered the most promising candidate for commercialization.

US 11,319,269 builds on the teachings that nickel-based catalysts are preferred hydrogenation catalysts to supplement tungsten in the catalyst systems. According to this patent a hydrogenation catalyst comprising nickel on a low surface area inert support should be used. The hydrogenation catalyst is a nickel-containing catalyst on a support with a surface area of less than 100 $m^2/g$, which contains less than 10 mass percent nickel, and which has a maximum particle dimension of less than 100 micrometers. The alleged advantage of such a catalyst system resides in that low concentrations of tungsten can be used with these hydrogenation catalysts to obtain high ethylene glycol selectivities. The patent does not provide evidence that such a goal has been achieved.

WO 2016/114661 discloses a continuous process for preparation of ethylene glycol from a carbohydrate source. Said process is carried out in a continuous stirred tank reactor (CSTR) in which a catalyst system is present. Said catalyst system comprises a tungsten compound and at least one hydrogenolysis metal. The hydrogenolysis metal is preferably present in the form of a catalyst supported on a support. Such heterogeneous catalyst particles can fairly easily be separated from the effluent stream, e.g. by a sieve plate, and added back. The tungsten compound on the other hand is generally present dissolved or dispersed in the liquid reaction medium (i.e. present as a homogenous catalyst compound) and not so easily removed from the effluent stream. Hence, the tungsten compound is partly removed as part of the effluent in operating the process in a CSTR. Examples show that the catalyst system used in the process according to WO2016/114661 loses its effectiveness rather quickly when a desirably higher concentration of carbohydrate is employed.

A low concentration of carbohydrate adds to the complexity and costs of the process, e.g., in the separation of the eventual product. Therefore, there is a need to maintain the effectiveness, more especially the selectivity, of a catalyst system comprising a tungsten compound and a heterogeneous catalyst.

**Summary of the invention**

It has now been found that, contrary to the teachings of US 11,319,269, other properties can be used to determine whether a catalyst system allows stable conversion of carbohydrate sources at high selectivity to ethylene glycol and/or propylene glycol. While heterogeneous catalysts comprising an active metal such as ruthenium on a support such as carbon were known and can be prepared by the person skilled in the art, we found how to select those which are suitable for use in converting carbohydrate sources at stable conversion and high selectivity to ethylene glycol and/or propylene glycol.

Accordingly, the present invention provides a process for the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from groups 8, 9 and 10 of the Periodic Table of the Elements, wherein the heterogeneous catalyst (a) has an active metal surface area (AMSA) of from 4.0 to 8.0 m$^2$/g, as determined by hydrogen chemisorption, (b) a ratio of percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0 and (c) $7.18 \times AMSA - \frac{\%(DA100-FP)}{AMSA}$ is at least 26.38.

Another embodiment of the invention resides in a process for the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from the metals of groups 8, 9 and 10 of the Periodic Table of the Elements, wherein the heterogeneous catalyst prior to employment of the heterogeneous catalyst in the process of reacting the carbohydrate source with hydrogen is first tested whether it fulfils the requirement that this heterogeneous catalyst has

(a) an active metal surface area (AMSA) of from 4.0 to 8.0 m$^2$/g, as determined by hydrogen chemisorption,
(b) a ratio of percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0, and

(c) $7.18 \times AMSA - \frac{\%(DA100-FP)}{AMSA}$
(c) is at least 26.38.

In a further embodiment the invention relates to a process for selecting a heterogeneous catalyst for use in the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from the metals of groups 8, 9 and 10 of the Periodic Table of the Elements, wherein a heterogeneous catalyst is selected which fulfils the requirement that the heterogeneous catalyst (i) has an active metal surface area (AMSA) of from 4.0 to 8.0 m$^2$/g, as determined by hydrogen chemisorption, (ii) a ratio of percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0 and (iii) $7.18 \times AMSA - \frac{\%(DA100-FP)}{AMSA}$ is at least 26.38.

## Detailed description of the invention

The catalyst system comprises a tungsten compound. This tungsten compound can be selected from a wide range of compounds. By the term "tungsten compound" is understood that tungsten is not present in its metallic form, hence not in its elemental state. The tungsten compound comprises tungsten bound to at least one other element. Such compounds may include tungsten carbide, nitride or phosphide.

Preferably, the tungsten compound has an oxidation state of at least +2, preferably having an oxidation state of +5 or +6. The tungsten compound is then suitably selected from the group consisting of tungstic acid ($H_2WO_4$), ammonium tungstate, ammonium metatungstate, ammonium paratungstate, tungstate compounds comprising at least one Group 1 or 2 element, metatungstate compounds comprising at least one Group 1 or 2 element, paratungstate compounds comprising at least one Group 1 or 2 element, tungsten oxide ($WO_3$), heteropoly compounds of tungsten, and combinations thereof. Preferred tungsten compounds are tungstic acid, tungstate compounds comprising at least one Group 1 or 2 element and combinations thereof. In a particularly preferred embodiment the tungsten compound comprises the reaction product of tungstic acid with an alkali metal-containing base, preferably being alkali metal hydroxide, more preferably being sodium hydroxide and/or potassium hydroxide.

Whereas the prior art has found that certain tungsten compounds leached from their supports and that such was considered a disadvantage, it has been found that it is advantageous to use catalyst systems that include tungsten compounds that dissolve in the reaction mixture. It has been found that the catalytic activity of the tungsten compound increases if the tungsten compound is dissolved. As explained in WO 2016/114661 it is believed that in the reducing atmosphere that is created in the reaction zone by means of the presence of hydrogen and carbohydrates, hexavalent tungsten compounds may be reduced to pentavalent tungsten and dissolve into the diluent. In this partly reduced state the tungsten ions are effective in attacking the carbon-carbon bonds in the carbohydrate source to form alkylene glycol precursors. It is therefore believed that when the catalyst system is used in the preparation of alkylene glycols from carbohydrate sources tungsten in various oxidation states are present.

Prior art documents have shown in the known processes according to the prior art the active metal of the heterogeneous catalyst can be selected from a wide range of metals. Accordingly, the active metal is selected from the groups 8, 9 and 10 of the Periodic Table of the Elements. The heterogeneous catalyst further comprises a support. The present inventors have found that the combination of active metal and support must have a certain active metal surface area to arrive at the desired ethylene glycol selectivity.

Hydrogen chemisorption measured on catalysts comprising metal and a support, is commonly used for the determination of metal specific surface area, but also the metal dispersion and metal particle size calculation. The active metal surface area of the catalyst can be determined by means of a hydrogen chemisorption isotherm. Conventional volumetric chemisorption techniques which are employed to measure hydrogen chemisorption are discussed in Structure of Metallic Catalysts, J. R. Anderson, Academic Press, 1975, Chapter 6. An automatic analysis program is used to measure a full isotherm of hydrogen. The active metal surface area was calculated using the equation:

$$AMSA = 6.023 \times 10^{23} \times V \times SF \times A/22414,$$

wherein

  AMSA is the active metal surface area;
  V is the uptake of hydrogen in ml/g;
  SF is Stoichiometry Factor;
  A is the area occupied by one atom of active metal in $nm^2$/atom.

This equation is disclosed by Micromeritics in Operators Manual for ASAP 2000 Chemi System V 1.00, Appendix C, Part No. 200-42808-01, 18 January 1991. Further details of the measurement of AMSA are provided in the examples.

The heterogeneous catalyst has an active metal surface area of from 4.0 to 8.0 $m^2$/g as determined by hydrogen chemisorption. The weight of catalyst (g) in the units for active metal surface area always refers to the dry catalyst weight.

The inventors of the present invention further found that a specific acidity of the heterogeneous catalyst relative to the AMSA has a significant impact on the performance of the catalyst system. This ratio is $\frac{\%(DA100-FP)}{AMSA}$ and will be discussed below. A further important parameter is 17.8 × AMSAvalue-this ratio.

The most common method to determine acidity is ammonia temperature programmed desorption (NH3-TPD). One of the reasons why NH3-TPD is used is that quantification results are reproducible. Further, the implementation of the experiment is relatively simple and acidic sites of different acid strength can be distinguished, i.e. weak, intermediate and strong acidic sites. Adsorbed ammonia cannot easily be desorbed from strong acidic sites. The way in which ammonia desorption is to be measured is described in the examples. It was found that it is advantageous that the heterogeneous catalyst comprises specific kind and amount of acidic sites with respect to the AMSA. This appears to be beneficial for the catalytic activity in the reaction of carbohydrates to alkylene glycols

It was found that for good performance, the heterogeneous catalyst has to have a ratio of the percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0. Details on how (% DA100-FP) is to be measured are described in the examples.

Furthermore, it was found that the AMSA and the (% DA100-FP) of the heterogeneous catalyst also have to be such that $7.18 \times AMSA - \frac{\%(DA100-FP)}{AMSA}$ is at least 26.38. The upper limit preferably is at most 80, more preferably at most 70.

Only when the heterogeneous catalyst fulfils all the requirements (a), (b) and (c), stable catalytic activity at high selectivity was observed. Hence, it is preferred that prior to conducting a process for the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from groups 8, 9 and 10 of the Periodic Table of the Elements, the heterogeneous catalyst is first subjected to the tests as herein described. This is preferably done per batch of catalyst that is used, as batches of catalyst of even a single supplier may have differences in the aspect that is tested on.

Furthermore, it is preferred that the heterogeneous catalyst fulfils the requirement (d) that there is a first peak of the ammonia temperature-programmed desorption (NH3-TPD) diagram wherein adsorbed ammonia is desorbed at increasing temperature, in the range of 90 to 180 °C. The first peak is to be measured as described above and in the examples.

Furthermore, the heterogeneous catalyst preferably fulfils the requirement (e) that the percentage of the adsorbed ammonia that is desorbed at the ammonia temperature-programmed desorption (NH3-TPD) at a temperature range of 100 °C to the temperature of the first peak (% DA100-FP), is at most 50.

Furthermore, it is preferred that the percentage of the area %DA100-FP is at least 1, more preferred at least 3, and most preferred at least 5.

Without wishing to be bound by any theory, it is believed that catalysts meeting the equation strike a nice balance between the metal hydrogenation function and the level of acidity. It is expected that when the acidity is too high the interaction between the tungsten compound and the active metal may become too intense so that the catalyst deactivates too quickly.

The weight ratio between active metal and support in the heterogeneous catalyst may vary within wide ranges. It is therefore feasible to have heterogeneous catalysts having an active metal to support weight ratio in the range of 100:100 to 1:100. As the pore sizes of support materials are easily adaptable to facilitate the access of reagent molecules to well-dispersed active metal, it is preferred to have a heterogeneous catalyst with an active metal to support weight ratio in the range of 20:100 to 1:100, more preferably from 10:100 to 2:100. In these weight ratios the active metal is calculated in its elemental form.

Contrary to the teachings of US 11,319,269 it is advantageous in the present invention to have a catalyst system wherein the support of the heterogeneous catalyst, and hence also the heterogeneous catalyst itself has a large surface area. As stated hereinbefore this allows easy access of any reagent of the envisaged reaction to the active sites on the active metal surface. Accordingly, the catalyst system comprises preferably an heterogeneous catalyst that has a BET surface area of at least 200 $m^2$/g, more preferably a surface area of at least 400 $m^2$/g, more preferably at least 400 $m^2$/g. The maximum surface area may be very large, e.g., up to 2000 $m^2$/g. However, from a practical point of view and also to enable catalysts with high mechanical strength the hydrogenolysis catalysts suitably have a surface area up to 1000 $m^2$/g. BET surface area can be measure for example byASTM D 5158-98.

Furthermore, it is preferred that the support of the heterogeneous catalyst has a particle size distribution according to ISO 13320-1 :2020 wherein D50 micrometer (average %) is 37.1-45.2 %. Preferably, it is further preferred that D0 micrometer (average %) is 1.7-1.9, D10 micron (average %) is 12.1-13.9 micrometer and D90 micrometer (average %) is 131.9-164.4. This means that is most preferred that substantially no particles fall through a sieve having openings of 1.7-1.9, 10 % fall through a sieve having openings of 12.1-13.9 micrometer, 50 % fall through a sieve having openings of 37.1-45.2 micrometer and 90 % fall through a sieve having openings of 131.9-164.4 micrometer. All support used in the current examples fulfil this requirement.

The active metals in the heterogeneous catalyst are selected from groups 8, 9 and 10 of the Periodic Table of the Elements. They may suitably be selected from the group consisting of Cu, Fe, Ni, Co, Pt, Pd, Ru, Rh, Ir, Os and combinations thereof. It is feasible that more than one active metal is used in the heterogeneous catalyst. Suitably, the combination of active metals comprises at least one metal selected from Pd, Pt, Ru, Rh and Ir in combination with another metal from groups 8, 9 or 10 of the Periodic Table of the Elements. The catalyst, suitably, comprises a mixture of two or more metals of the group consisting of Ru, Pt, Pd, Ir and Rh. Suitable examples are Ru/Ir, Ru/Pt, Ru/Pd. When two metals are used, the weight ratio is suitably in the range of 0.1 : 1 to 20: 1, preferably in the range of 0.5: 1 to 10: 1. The active metal or metals may suitably be present in its or their metallic form or as its or their hydride or oxide. Advantageously, the active metals will be reduced to their metallic form prior to exposure to the reaction. Alternatively, the metal compounds will be exposed to the reaction in the crude form, whereafter they will be reduced during the reaction in the presence of hydrogen.

The heterogeneous catalyst comprises an active metal or a combination of active metals supported on a support. The support may be selected from a wide range of known supports. Suitable supports include activated carbon, silica, zirconia, alumina, silica-alumina, titania, niobia, iron oxide, tin oxide, zinc oxide, silica-zirconia, zeolitic aluminosilicates, titanosilicates, magnesia, silicon carbide, clays and combinations thereof. The skilled person will know that activated carbon is an amorphous form of carbon with a surface area of up to 1000 $m^2$/g. Such activated carbon thus has a porous structure. Most preferred supports are activated carbon, silica, silica-alumina and alumina, since excellent results have been obtained therewith. More preferably, the heterogeneous catalyst comprises ruthenium as the active metal and activated carbon as the support.

The ratio between the active metal or metal combination on the heterogeneous catalyst and the tungsten compound may vary between wide ranges. In accordance with prior art teachings the weight ratio between these components may vary from 0.02 to 3000. In the present invention the molar ratio of tungsten to active metal on the heterogeneous catalyst is preferably in the range of 1 to 25. Preferably, the molar ratio of tungsten to active metal is in the range of 2 to 20, more preferably from 5 to 15. If the ratio is beyond the limits of these ranges, the relative yield of alkylene glycols other than ethylene glycol is decreased and/or the conversion of the carbohydrate is slowed down. Expressed in weight, the weight ratio of tungsten compound, calculated as tungsten metal, to active metal on heterogeneous catalyst, calculated as active metal, is preferably in the range of 2 to 50 wt/wt, more preferably from 5 to 50 wt/wt.

It is commonly known that many metals of the Groups 8, 9 and 10 of the Periodic Table of the Elements are used in the preparation of heterogeneous hydrogenation catalysts for a variety of chemical reactions involving hydrogen. The use of these metals is based on their ability to dissociate and thereby activate molecular hydrogen, providing hydrogen atoms to react with the organic species, themselves activated through interaction with sites at the catalyst surface. The heterogeneous catalyst of the present invention may be selected from such conventional hydrogenation catalysts. The heterogeneous catalyst may also be prepared by conventional methods. The conventional hydrogenation catalyst providers/suppliers, however, are silent on the properties that have been found crucial in the present invention. Before using the heterogeneous catalyst in a catalyst system according to the present invention it is therefore preferable to analyze the heterogeneous catalyst to verify whether it fulfils the requirements (a), (b) and (c), and preferably one or more of (d), (e) and (f) before it is used in the catalyst system for the preparation of ethylene glycol and/or propylene glycol from a carbohydrate source by reacting the carbohydrate source with hydrogen in the presence of the catalyst system.

The carbohydrate source can be selected from a variety of sources. Suitably, the carbohydrate source comprises or consists of a carbohydrate selected from the group consisting of polysaccharides, oligosaccharides, disaccharides, and monosaccharides.

Suitable examples include sustainable sources such as cellulose, hemicellulose, starch, partially hydrolyzed starch, sugars, such as sucrose, mannose, arabinose, glucose, fructose and mixtures thereof. Sources that contain the above carbohydrates may include dextrose syrups, maltose syrups, sucrose syrups, glucose syrups, crystalline sucrose, crystalline glucose, wheat starch, corn starch, potato starch, cassava starch, and other carbohydrate-containing streams such as paper pulp streams, municipal waste water streams and other glucose units-containing streams can be used as well, for example from wood waste, paper waste, agricultural waste, municipal waste, paper, cardboard, sugar cane, sugar beet, wheat, rye, barley, other agricultural crops and combinations thereof. These streams may require pre-treatment to remove components that interfere with the current process such as basic fillers, e.g. calcium carbonate in waste paper. In this way the process according to the invention may not only be used from natural sources, but can even be used to upgrade and usefully re-use waste streams. Preferably, the carbohydrate in the carbohydrate source is selected from the group consisting of cellulose, starch, partially hydrolyzed starch, glucose, sucrose, glucose-oligomers, paper waste, and combinations thereof, more preferably glucose, sucrose or starch. Since cellulose presents difficulties that are absent in other carbohydrate sources, the carbohydrate source is even more preferably selected from the group consisting of starch, partially hydrolyzed starch, hemicelluloses and hemicellulose sugars, glucose, sucrose and combinations thereof, and is most preferably selected from glucose, sucrose and combinations thereof.

A carbohydrate feed is prepared by dissolving the carbohydrate source in a solvent. For reasons of e.g. cost and solubility, it is preferred that the solvent in which the carbohydrate feed of the carbohydrate source is dissolved comprises water and, optionally, one or more alkylene glycols, and preferably is water. The concentration of carbohydrate source in the solvent is preferably at least 1.0 wt. % based on the weight of carbohydrate source and solvent, preferably at least 2.0 wt. %, more preferably at least 5.0 wt. %, even more preferably at least 10.0 wt. %, and still more preferably at least 20.0 wt. %. The carbohydrate source in the solvent is preferably at most 90.0 wt. %, based on the weight of carbohydrate source and solvent, more preferably at most 70.0 wt. % and even more preferably at most 50.0 wt. %. A feed stream containing carbohydrate source within this concentration range can suitably be easily transported. The feed stream can also consist of only (100 wt. %) carbohydrate source.

The preparation of ethylene glycol and/or propylene glycol from a carbohydrate source has been described in several documents, some of which have been described in the background of the present invention. Many variants of the process use a tungsten compound as a so-called retro-aldol catalyst and a heterogeneous catalyst comprising an active metal and a support. The tungsten compound is commonly at least party dissolved in the reaction medium, whereas the heterogeneous catalyst is a solid, and thus heterogeneous catalyst. As described in WO2016/144661 the process is suitably conducted as a continuous process in a reactor, preferably in a continuous stirred tank reactor.

The catalyst system according to the present invention is preferably added to the reaction via a method wherein a first catalyst feed that comprises the tungsten compound in a liquid in which it is at least partly dissolved is prepared,

wherein a second catalyst feed that comprises the heterogeneous catalyst is prepared,
wherein the carbohydrate source and hydrogen are fed to a heated and pressurized reactor into which also the first catalyst feed and at least a one dose of the second catalyst feed are fed, and
wherein further amounts of second catalyst feed, as single dose, in multiple doses, or continuously are provided.

The first catalyst feed preferably contains tungstic acid, an alkali metal hydroxide and an alkylene glycol-comprising liquid to form a homogeneous first catalyst feed.

Preferably, the heterogeneous catalyst comprising a support and an active metal selected from groups 8, 9 and 10 of the Periodic Table of the Elements is subjected to hydrogen chemisorption and ammonia temperature-programmed desorption (NH3-TPD) to determine the AMSA and %DA100-FP as described above after which it is assessed whether the heterogeneous catalyst fulfils the requirements (a), (b) and (c) and optionally furthermore one or more of (d), (e) and (f). After this assessment, the heterogeneous catalyst can be contacted with a carbohydrate source and hydrogen.

In a continuous process it is advantageous to add tungsten compound continuously or periodically to the reactor. Since in a continuous process product is continuously withdrawn from the reactor and therefore also the homogeneous component of the catalyst system, i.e., the tungsten compound, is withdrawn with the product, it is preferred to add tungsten compound. Addition of tungsten compound may be periodically or continuously. It is advantageous to ensure that the amount of tungsten compound added to the reactor is such that the concentration thereof in the reactor is substantially constant.

The concentration of tungsten compound, calculated as tungsten metal, based on the weight of carbohydrate source introduced into the reactor, is preferably in the range of 0.1 to 50 wt. %, more preferably in the range of 1 wt. % 35 wt. %. Even more preferably the concentration of tungsten compound, calculated as tungsten metal, based on the weight of carbohydrate source introduced into the reactor, is preferably in the range of 0.2 wt. % to 25 wt. %, even more preferably from 2 wt. % to 25 wt. %.

Although the present catalyst system shows a remarkably stable performance, it is possible that some deterioration of the performance of the heterogeneous catalyst may occur. It may therefore be advantageous to continuously or periodically add heterogeneous catalyst to the reactor. At the same time a portion of the heterogeneous catalyst that has been present in the reactor and that shows a reduced catalytic activity may be removed. The heterogeneous catalyst can be separated off and regenerated and recycled, as is set out e.g. in WO 2019/175365. Preferably, the amount of heterogeneous catalyst that is added to the reactor is such that the concentration thereof in the reactor is substantially constant.

The ratio between active metal on the heterogeneous catalyst and the tungsten compound may vary between wide ranges. As stated hereinabove, the weight ratio between these components may vary from 0.02 to 3000 according to prior art teachings. In the present invention the molar ratio of tungsten to active metal on the heterogeneous catalyst is preferably in the range of 1 to 25. Preferably, the molar ratio of tungsten to active metal is in the range of 2 to 20, more preferably from 5 to 15. Expressed in weight, the weight ratio of tungsten compound, calculated as tungsten metal, to active metal on heterogeneous catalyst, calculated as active metal, is preferably in the range of 2 to 50 wt/wt, more preferably from 5 to 50 wt/wt. In terms of the concentration of active metal with relation to carbohydrate source, it is preferable that the weight ratio of heterogeneous catalyst, calculated as active metal, to carbohydrate that is introduced into the reactor is in the range of 1:25 to 1:250 wt/wt, more preferably from 1:50 to 1:200 wt/wt.

The residence time in the reactor may vary. Preferably the mean residence time of the carbohydrate source in the reactor is at least 1 min. (By mean residence time is herein understood the average time spent by a material flowing at a volumetric rate "u" through a volume "V", as further explained in the handbook "Modeling of Chemical Kinetics and Reactor Design" by A. Kayode Coker, published in 2001 by Butterworth Heinemann). Preferably the mean residence time of the carbohydrate source is in the range from equal to or more than 1 minutes to equal to or less than 6 hours, more preferably from equal to or more than 3 minutes to 2 hours, most preferable in the range from equal to or more than 5 minutes to equal to or less than 45 minutes. If the carbohydrate source reacts quickly, however, the mean residence time may also be shorter than 5 minutes and even shorter than 3 minutes.

Preferably the process is a continuous process. Preferably a continuous process is operated at a weight hourly space velocity (WHSV), expressed as the mass of carbohydrate source per mass of heterogeneous catalyst, expressed as active metal, per hour, in the range of 0.01 to 100 $hr^{-1}$, preferably from 0.01 to 10 $hr^{-1}$, more specifically of from 0.02 to 5 $hr^{-1}$, more specifically of from 0.05 to 2 $hr^{-1}$.

The hydrogen partial pressure applied during step (i) preferably lies in the range from equal to or more than 1.0 MPa, preferably equal to or more than 2.0 MPa, more preferably equal to or more than 3.0 MPa to equal to or less than 16.0 MPa, preferably equal to or less than 12.0 MPa, more preferably equal to or less than 8.0 MPa. All pressures herein are absolute pressures. The total pressure applied during the reaction is suitably at least 1.0 MPa, preferably at least 2.0 MPa, more preferably at least 3.0 MPa. The total pressure applied during the reaction is suitably at most 16.0 MPa, more preferably at most 10.0 MPa. Preferably the reactor is pressurized with hydrogen before addition of any starting material. The person skilled in the art will understand that the pressure at 20°C will be lower than the actual pressure at the reaction temperature. The pressure applied during the reaction when converted back to 20°C, preferably equals a pressure in the range from equal to or more than 0.7 MPa to equal to or less than 8.0 MPa.

If the process is a continuous or semi-continuous process, the hydrogen is preferably supplied in a continuous or semi-continuous manner. In the reactor at least a portion of the carbohydrate source is reacted in the presence of the hydrogen, at a temperature in the range from 170°C to 270°C, more preferably at a temperature in the range from 200°C to 250°C. The reactor may be brought to a temperature within these ranges before addition of any starting material and can be maintained at a temperature within the range. As will be understood by the person skilled in the art, to start the reaction to form ethylene glycol in the reactor carbohydrate feed, hydrogen, and both catalysts need to be present at the right temperature and pressure.

The heterogeneous catalyst preferably is a hydrogenolysis catalyst.

As mentioned above, the heterogeneous catalyst can be obtained or prepared in anyway. It may be selected from conventional or commercial heterogeneous catalysts. We have found a way to select those catalysts which are suitable for the present specific application. This allows to verify whether the catalyst fulfils the requirements for use as catalyst system according to the invention.

Preferably, a heterogeneous catalyst is selected which fulfils the requirement that the heterogeneous catalyst has (iv) a first peak in the ammonia temperature-programmed desorption (NH3-TPD) diagram in the range of 90 to 180 °C

A heterogeneous catalyst preferably is selected which fulfils the requirement that (v) the % DA100-FP is at most 50.

The heterogeneous catalyst preferably further is selected because it fulfils the requirement that (v) the heterogeneous catalyst has a BET surface area in the range of 400 to 1000 $m^2/g$.

**EXAMPLES**

Process description

Hydrogenolysis experiments were carried out in a 200 ml-continuous stirred tank reactor provided with a radial flow impeller as stirrer. The amount of liquid in the reactor was about 145 $\pm$ 3 ml. Experiments were conducted with a 30 minutes residence time.

The reactor contained as heterogeneous catalyst ruthenium on activated carbon (Ru on AC). The amount of ruthenium on activated carbon was about 5 wt% Ru on AC. The total weight of heterogeneous catalyst added to the reactor before the experiment began as a pre-fill was about 39.6 g/L Ru on AC (dry). The reactor was filled with the pre-fill Ru on AC and water before the reactor was heated (to 230°C) and pressurized to 65 bar.g. All of the heterogeneous catalyst remained in the reactor during the reaction. Liquid product and effluent gas were collected continuously from the reactor through a 20 micron dead-end filter which is set at the reactor level.

A feed solution containing carbohydrate (sucrose) and tungstic acid ($H_2WO_4$) was prepared by dissolving sucrose in water, and adding some ethylene glycol to it. Separately, tungstic acid was dissolved in ethylene glycol (in an amount of about 5% by weight). These two solutions were mixed, to arrive at a combined feed composition containing: sucrose at a concentration of about 25 wt%, and about 55.5 wt% water and about 19 wt% ethylene glycol (the remainder mainly being tungstic acid and some sodium hydroxide). Then sodium hydroxide was added, in a molar ratio sodium:

tungsten of 0.75.

The reactor was heated to 230°C and pressurized with hydrogen gas to 6.5 MPa. Hydrogen gas was fed into the reactor at a flow of about 6760 ml/minute/liter reactor volume.

At the start of the reaction (t = 0 hours) the feed solution with sucrose and tungsten compound was pumped into the reactor at a steady flow to obtain a residence time of 30 minutes (at flow rate of 33.1 ml/minute/liter reactor volume).

Reactions were carried out for about 300 minutes. Samples after about 240 and 300 minutes were used for analysis by liquid chromatography and the selectivities to ethylene glycol (EG), propylene glycol (PG) and butylene glycol (BG) have been reported.

$$Selectivity\ X = \frac{Concentration\ X\ in\ Product - Concentration\ X\ in\ Feed}{Concentration\ Sucrose\ in\ feed - Concentration\ Sucrose\ in\ product} -$$

where concentration sucrose in product after 30 mins approaches zero.

Heterogeneous catalysts

Ten commercial hydrogenation catalysts of ruthenium on activated carbon (Ru/C) with a nominal ruthenium content of 5 wt.%, were analyzed. Their NH3-TPD graphs, their active metal surface areas, their particle size distribution and their BET surface areas were determined.

Particle Size Distribution

The particle size distribution of the heterogeneous catalysts was assessed according to ISO 13320-1 :2020. All samples fulfil the following particle size distribution in that the D0 micrometer (average %) is 1.7-1.9, D10 micron (average %) is 12.1-13.9 micrometer, D50 micrometer (average %) is 37.1-45.2 % and D90 micrometer (average %) is 131.9-164.4.

Acid sites via NH3-TPD determination

A sample of each catalyst was held in a quartz reactor and placed into a Micromeritics TPD/TPR 2900 analyzer. In the analyzer the sample was subjected to a temperature program from 20 °C to 550 °C in helium at a heating rate of 10 °C/min. The sample was held at 550 °C for 30 min in a flow of helium. Subsequently, the sample was cooled down to 40 °C. Then the sample was flushed alternately with ammonia for 10 min and helium for 10 min. This procedure was repeated three times. After settling down after the last flushing step the desorption of ammonia was monitored in a temperature range of 40 to 550 °C at a ramp rate of 10 °C/min. The relative amount of desorbed ammonia was calculated as the area under the graph from 100 °C to the temperature of the first peak in relation to the total area under the entire graph from 100 to 550°C.

Active metal surface area via Hydrogen Chemisorption

A sample of the Ru/C catalyst was dried under vacuum at 105 °C. The dried sample was subjected to reduction with hydrogen at 230 °C for two hours. Subsequently, the sample was cooled down to 40 °C under vacuum. Then the hydrogen adsorption isotherm on the thus activated catalyst was determined. For the calculation of the active metal surface area the above-mentioned formula as used:

$$S.A = 6.023 \times 10^{23} \times V \times SF \times A/22414,$$

wherein

S.A. is the active metal surface area;
V is the uptake of hydrogen in ml/g;
SF is Stoichiometry Factor;
A is the area occupied by one atom of active metal in nm$^2$/atom.

The stoichiometry factor was 2; for the surface area of one Ru atom the A value 0.06135 nm$^2$/atom was used.

BET surface area by N2 adsorption

The adsorption analysis with N2 as adsorptive was recorded at 77 K on a Micromeritics Tristar II 3020. A relative pressure range from p/p0 = 0.01 up to p/p0 = 0.99 was applied. Prior to the actual adsorption investigations, the samples have been pre-treated at 100°C in vacuum for 16 hours. The dry sample mass obtained after the pre-treatment has been used in the various calculations according to ISO 9277:2010.2. The results of the analyses for the ten tested catalysts are shown in Table 1.

Table 1 - Parameters of heterogeneous catalysts

| Catalyst No. | %DA100-FP | first NH3-TPD peak, °C | AMSA, $m^2$/g | (%DA100-FP)/AMSA | 7.18 xAMSA $-\dfrac{\%(DA100-FP)}{AMSA}$ | BET surface area, $m^2$/g |
|---|---|---|---|---|---|---|
| 1 | 35 | 178.19 | 5.3 | 6.6 | 31.45 | - |
| 2 | 6 | 115.03 | 7.7 | 0.8 | 54.49 | 717 |
| 3 | 31 | 161.27 | 4.7 | 6.6 | 27.15 | 760 |
| 4 | 10 | 130.14 | 6.4 | 1.6 | 44.35 | 782 |
| 5 | 33 | 183.6 | 5.6 | 5.9 | 34.31 | 795 |
| 6 | 5 | 116.72 | 4.3 | 1.2 | 29.67 | - |
| 7 | 4 | 115.14 | 3.3 | 1.2 | 22.49 | - |
| 8 | 32 | 192.28 | 2.9 | 11.1 | 9.72 | 761 |
| 9 | 4 | 118.72 | 3.7 | 1.1 | 25.47 | 855 |
| 10 | 34 | 168.15 | 4.6 | 7.4 | 25.63 | 811 |

Results

The ten heterogeneous catalysts together with tungstic acid/sodium tungstate were tested in the conversion of sucrose to alkylene glycols in a continuous process as described above. The selectivities (in mass percentages) for the three most prominent alkylene glycols were determined after about 3, 4 and 5 hours. The most preferred alkylene glycols are ethylene glycol (EG) and propylene glycol (PG), the least preferred glycol is 1,2-butanediol (BG). The results are shown in Table 2.

Table 2 - Selectivity to various alkylene glycols

| Catalyst No. | Reaction time, min | Selectivity EG, % | Selectivity PG, % | Selectivity BG, % |
|---|---|---|---|---|
| 1 | 183 | 28,19 | 20.35 | 1.39 |
| | 240 | 35.61 | 21.17 | 2.00 |
| | 300 | 40.63 | 21.74 | 1.91 |
| 2 | 180 | 27.65 | 22.05 | 1.39 |
| | 241 | 35.08 | 22.48 | 1.54 |
| | 300 | 38.89 | 22.63 | 1.59 |
| 3 | 186 | 31.88 | 23.07 | 1.28 |
| | 242 | 35.33 | 22.93 | 1.55 |
| | 301 | 38.12 | 23.24 | 1.76 |
| 4 | 180 | 30.49 | 20.49 | 1.43 |
| | 240 | 39.52 | 21.39 | 1.63 |
| | 300 | 40.02 | 20.98 | 1.81 |
| 5 | 180 | 32.54 | 22.77 | 1.85 |
| | 240 | 32.83 | 22.92 | 1.98 |
| | 300 | 34.72 | 23.51 | 2.11 |

(continued)

| Catalyst No. | Reaction time, min | Selectivity EG, % | Selectivity PG, % | Selectivity BG, % |
|---|---|---|---|---|
| 6 | 187 | 35.64 | 23.36 | 1.57 |
|  | 239 | 40.85 | 23.32 | 2.08 |
|  | 301 | 41.29 | 23.08 | 2.64 |
| 7 | 120 | 32.73 | 19.52 | 4.15 |
|  | 150 | 30.73 | 18.25 | 5.06 |
| 8 | 180 | 34.22 | 24.47 | 3.97 |
|  | 240 | 30.40 | 23.20 | 4.75 |
|  | 300 | 28.06 | 22,75 | 4.55 |
| 9 | 180 | 39.76 | 24.36 | 3.58 |
|  | 240 | 36.96 | 23.43 | 4.44 |
|  | 300 | 34.24 | 23.04 | 4.45 |
| 10 | 180 | 29.33 | 23.10 | 2.74 |
|  | 240 | 30.14 | 22.44 | 3.36 |
|  | 304 | 26.82 | 21.87 | 3.53 |

The results show that catalyst systems 1 to 6 that are according to the present invention provide a stable performance after a reaction period of five hours. They do not only result in high selectivity for ethylene glycol, viz. above 35 %, and even above 40 % but also increase the selectivity to ethylene glycol in the fourth to fifth hour of reaction.

The heterogeneous catalyst of comparative catalyst systems 7 to 9 do not fulfil requirements a) and c) while system 8 additionally also does not fulfil requirement b). The performance of catalyst 7 already worsens after two hours, whereas catalyst systems 8 and 9 show a decline in the EG selectivity after three and four hours.

Catalyst 10 does not fulfil requirement c). The performance of this catalyst system for the preparation of alkylene glycols yields a selectivity to ethylene glycol below 35% and a relatively high selectivity to undesired butylene glycol. Also the performance worsens after four hours.

## Claims

1. Process for the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from groups 8, 9 and 10 of the Periodic Table of the Elements, wherein the heterogeneous catalyst has

   (a) an active metal surface area (AMSA) of from 4.0 to 8.0 $m^2/g$, as determined by hydrogen chemisorption,
   (b) a ratio of percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0, and

   (c) $7.18 \times AMSA - \frac{\%(DA100-FP)}{AMSA}$ is at least 26.38.

2. Process for the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from the metals of groups 8, 9 and 10 of the Periodic Table of the Elements, wherein the heterogeneous catalyst prior to employment of the heterogeneous catalyst in the process of reacting the carbohydrate source with hydrogen is first tested whether it fulfils the requirement that this heterogeneous catalyst has

(a) an active metal surface area (AMSA) of from 4.0 to 8.0 m$^2$/g, as determined by hydrogen chemisorption,
(b) a ratio of percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0, and

(c) $7.18 \times AMSA - \frac{\%(DA100-FP)}{AMSA}$ is at least 26.38.

3. Process according to claim 1 or 2, wherein the heterogeneous catalyst further has (d) a first peak in the ammonia temperature-programmed desorption (NH3-TPD) diagram in the range of 90 to 180 °C.

4. Process according to any of the preceding claims, wherein the heterogeneous catalyst further has (e) a % DA100-FP of at most 50.

5. Process according to any one of the preceding claims, wherein the heterogeneous catalyst further has (f) a BET surface area according to ISO 9277:2010.2 in the range of 400 to 1000 m$^2$/g.

6. Process according to any one of the preceding claims, wherein the heterogeneous catalyst comprises an active metal selected from the groups 8, 9 and 10 of the Periodic Table of the Elements in the metallic form.

7. Process according to any one of the preceding claims, wherein the heterogeneous catalyst comprises a support consisting of carbon, preferably the heterogeneous catalyst comprises ruthenium on activated carbon.

8. Process according to any one of the preceding claims, wherein tungsten compound comprises the reaction product of tungstic acid with an alkali metal-containing base, preferably being alkali metal hydroxide, more preferably being sodium hydroxide and/or potassium hydroxide.

9. Process according to any one of the preceding claims, wherein the carbohydrate is selected from the group consisting of cellulose, starch, glucose, sucrose, fructose, glucose-oligomers, paper waste, and combinations thereof, more preferably glucose, sucrose, starch and combinations thereof, most preferably glucose, sucrose and combinations thereof.

10. Process for selecting a heterogeneous catalyst for use in the preparation of ethylene glycol and/or propylene glycol from a carbohydrate source by reacting the carbohydrate source with hydrogen in the presence of a catalyst system which catalyst system comprises a tungsten compound and a heterogeneous catalyst comprising a support and an active metal which active metal is selected from the metals of groups 8, 9 and 10 of the Periodic Table of the Elements, wherein a heterogeneous catalyst is selected which fulfils the requirement that the heterogeneous catalyst has

(i) an active metal surface area (AMSA) of from 4.0 to 8.0 m$^2$/g, as determined by hydrogen chemisorption,
(ii) a ratio of percentage of the adsorbed ammonia that is desorbed at the ammonia temperature-programmed desorption (NH3-TPD) at a temperature range of 100 °C to the temperature of the first peak (% DA100-FP) to the AMSA ($\frac{\%(DA100-FP)}{AMSA}$) of from 0.35 to 8.0, and

(iii) $7.18 \times AMSA - \frac{\%(DA100-FP)}{AMSA}$ is at least 26.38.

11. Process according to claim 10, wherein the heterogeneous catalyst is selected which fulfils the requirement that the heterogeneous catalyst has (iv) a first peak in the ammonia temperature-programmed desorption (NH3-TPD) diagram in the range of 90 to 180 °C.

12. Process according to claim 10 or 11, wherein the heterogeneous catalyst further fulfils the requirement that (v) the % DA100-FP is at most 50.

13. Process according to any on of claims 10-12, wherein the heterogeneous catalyst further fulfils the requirement that (vi) the heterogeneous catalyst has a BET surface area according to ISO 9277:2010.2 in the range of 400 to 1000 m$^2$/g.

14. Process comprising subjecting heterogeneous catalyst comprising a support and an active metal selected from groups 8, 9 and 10 of the Periodic Table of the Elements to hydrogen chemisorption and ammonia temperature-programmed desorption (NH3-TPD) to determine the active metal surface area (AMSA) and the percentage of the adsorbed ammonia that is desorbed by NH3-TPD at a temperature range of 100 °C to the temperature of the first peak (% DA100-FP) and assess whether the heterogeneous catalyst fulfils the requirements (a), (b) and (c) and optionally furthermore one or more of (d), (e) and (f), all as defined in claims 1-4.

15. Process according to claim 14, wherein the heterogeneous catalyst is contacted with a carbohydrate source and hydrogen after the assessment.

Europäisches
Patentamt

European
Patent Office

Office européen
des brevets

# EUROPEAN SEARCH REPORT

**Application Number**

EP 24 19 8269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/002259 A1 (VAN DER WAAL JAN CORNELIS [NL] ET AL) 4 January 2018 (2018-01-04) | 1-9 | INV.<br>C07C29/132<br>C07C31/20<br>B01J23/46<br>C07C29/60 |
| A | * paragraph [0019] *<br>* examples 1,2 *<br>* table 1 *<br>* claims 1-11 * | 10-15 | |
| X | US 2021/040022 A1 (DEKKER PAULA [NL] ET AL) 11 February 2021 (2021-02-11) | 1-9 | |
| A | * paragraph [0058] *<br>* example 2 * | 10-15 | |

**TECHNICAL FIELDS SEARCHED (IPC)**

C07C
B01J

The present search report has been drawn up for all claims

| Place of search | Date of completion of the search | Examiner |
|---|---|---|
| The Hague | 22 January 2025 | Delanghe, Patrick |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 19 8269

This annex lists the patent family members relating to the patent documents cited in the above-mentioned European search report.
The members are as contained in the European Patent Office EDP file on
The European Patent Office is in no way liable for these particulars which are merely given for the purpose of information.

22-01-2025

| Patent document cited in search report | Publication date | Patent family member(s) | Publication date |
|---|---|---|---|
| US 2018002259 A1 | 04-01-2018 | AU 2016207242 A1 | 03-08-2017 |
| | | BR 112017014945 A2 | 13-03-2018 |
| | | CA 2973556 A1 | 21-07-2016 |
| | | CN 107406358 A | 28-11-2017 |
| | | EP 3245181 A1 | 22-11-2017 |
| | | JP 6553743 B2 | 31-07-2019 |
| | | JP 2018501321 A | 18-01-2018 |
| | | KR 20170105063 A | 18-09-2017 |
| | | LT 3245181 T | 25-02-2020 |
| | | MY 185257 A | 30-04-2021 |
| | | PL 3245181 T3 | 29-06-2020 |
| | | SG 11201705686R A | 30-08-2017 |
| | | UA 118147 C2 | 26-11-2018 |
| | | US 2018002259 A1 | 04-01-2018 |
| | | WO 2016114661 A1 | 21-07-2016 |
| US 2021040022 A1 | 11-02-2021 | BR 112020016904 A2 | 15-12-2020 |
| | | CN 111868018 A | 30-10-2020 |
| | | EA 202092099 A1 | 26-11-2020 |
| | | EP 3765433 A1 | 20-01-2021 |
| | | LT 3765433 T | 25-04-2024 |
| | | PL 3765433 T3 | 27-05-2024 |
| | | US 2021040022 A1 | 11-02-2021 |
| | | WO 2019175369 A1 | 19-09-2019 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7960594 A
- US 20110312488 A
- US 11319269 B

- WO 2016114661 A
- WO 2016144661 A
- WO 2019175365 A


**Non-patent literature cited in the description**

- **NA JI et al.** *ChemSusChem*, 2012, vol. 5, 939-944
- **M. ZHENG et al.** *Chin. J. Catal.*, 2014, vol. 35, 602-613
- **J. R. ANDERSON**. Structure of Metallic Catalysts. Academic Press, 1975

- *Micromeritics in Operators Manual for ASAP 2000 Chemi System*, 18 January 1991
- **A. KAYODE COKER**. Modeling of Chemical Kinetics and Reactor Design. Butterworth Heinemann, 2001